Europäisches Patentamt

European Patent Office

Office européen des brevets

(19)

(11) Publication number: **0 186 100 B1**

# EUROPEAN PATENT SPECIFICATION

(12)

(45) Date of publication of patent specification: **01.04.92**  (51) Int. Cl.⁵: **G01N 33/543**, G01N 33/552

(21) Application number: **85116087.9**

(22) Date of filing: **17.12.85**

(54) **Analytical device and method for using same.**

(30) Priority: **24.12.84 US 685565**

(43) Date of publication of application:
**02.07.86 Bulletin 86/27**

(45) Publication of the grant of the patent:
**01.04.92 Bulletin 92/14**

(84) Designated Contracting States:
**BE CH DE FR GB IT LI NL SE**

(56) References cited:
**EP-A- 0 112 721
EP-A- 0 197 784
WO-A-83/00391
DE-A- 3 045 199
US-A- 4 363 634**

(73) Proprietor: **ABBOTT LABORATORIES
14th Street and Sheridan Road North St
North Chicago, Illinois 60064(US)**

(72) Inventor: **Tan Yue, Vincent
430 Catalpa Lane
Libertyville, IL 60048(US)**
Inventor: **Houseman, Kenneth R.
309 Ahwahnee Lane
Lake Forest, IL 60048(US)**
Inventor: **Parsons, Robert George
419 Thornapple Lane
Libertyville, IL 60048(US)**

(74) Representative: **Modiano, Guido et al
MODIANO, JOSIF, PISANTY & STAUB
Modiano & Associati Via Meravigli, 16
I-20123 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**Description**

Background of the Invention

Technical Field

This invention relates generally to analytical devices and methods, and more particularly relates to an improved solid-phase analytical device and method for conducting assays of fluids utilizing the device, especially enzyme immunoassays of biological fluids such as blood, serum, plasma, urine, spinal fluid and the like.

Background Art

Various analytical procedures and devices are commonly employed in the testing of fluids for analytes, i.e., substances of interest or clinical significance which may be present therein. Particularly with respect to the diagnosis and treatment of disease or other conditions of the human body, the accurate determination, on a timely basis, of the presence or level of certain analytes in body fluids which are of clinical significance can have a profound influence on the ability of health care professionals to treat and manage disorders such as pathological physical conditions and disease states.

One assay methodology which has seen increasing application in the diagnosis of various disorders of the human body is the enzyme immunoassay (EIA). EIA techniques take advantage of the mechanisms of the immune systems of higher organisms, wherein antibodies are produced in response to the presence of substances (i.e., antigens) which are pathogenic or foreign to the organisms; one or more specific antibodies are produced in response to and are capable of reacting with a particular antigen, thereby making their reaction highly specific. Most typical EIA procedures involve a series of wet chemistry steps using liquid reagents, wherein the substance of interest (analyte) in a biological fluid under assay, e.g., an antigen or antibody in a test sample of whole blood or serum, is detected by initially causing such analyte in the sample to become bound to a corresponding antigen or antibody reagent which is introduced into the sample. Then, in a typical case where a so-called "sandwich" assay is performed, after unbound material has been removed, another antigen or antibody is introduced. This second antigen or antibody, however, is one which has been labeled or conjugated with an enzyme or other substance capable of producing or causing, when reacted with another suitable reagent such as a chromogen or dye, a detectable response such as color development. The detectable response so produced can then be read and interpreted, visually or instrumentally, as an indication or measure of the presence or amount of the antigen or antibody present in the original sample.

Solid-phase EIA procedures are generally considered preferable for both antibody and antigen assays because of their safety, ease of use, specificity and sensitivity by comparison with heretofore employed techniques such as the wet chemistry assays or radioimmunoassays (RIA). Moreover, the possibility that such solid-phase assays can be easily read instrumentally, such as by measuring color development by a spectrophotometer, is a feature of solid-phase EIA techniques which has resulted in their wide-spread use.

Thus, in one type of conventional solid-phase EIA "sandwich" assay, a test sample suspected of containing an antibody or antigen of interest is typically contacted by a plastic or glass bead or other support material which has been previously coated over its surface with a protein or another substance capable of retaining the antigen or antibody, either by immobilization thereof on the surface or by chemical binding. The coated support material, with the antigen or antibody from the sample thereupon, is then washed to remove unbound material; an antibody or antigen in the sample which is not retained an the support is thus removed. Thereafter, a second antigen or antibody, which may be conjugated (i.e., linked chemically) with an enzyme, is added and becomes bound to its corresponding antibody or antigen on the support. Following further washing to remove unbound material, a chromogen, for example, a benzidine-type indicator reactive in the presence of the enzyme, is then added and, because of its sensitivity to the presence of the enzyme, produces a color response which can be determined visually or instrumentally.

Known from US-A-4 363 634 is a device for use in a bioprocess comprising a transparent glass solid support, which is coated with a water-based synthetic polymer film, to which one or more functional biomaterials are adsorbed or covalently bonded.

Such assay techniques, and the use of the solid-phase bead or other types of supports for retaining antigen or antibody thereon in such assays, are well known and used, but have not been without drawbacks. For example, the necessity of elaborate apparatus for conducting the assay and containing the liquid reagents employed in such assays often results in substantial labor and equipment costs, especially for Low-volume, individual sample testing. Moreover, the accuracy and reproducibility of such assays can often be less than optimum, since it is sometimes difficult to manufacture conventional coated solid supports and other apparatus associated with such assays so that for a particular assay all of the materials used therein are specifically designed to meet sensitivity and specificity requirements.

## Summary of the Invention

The present invention provides an improved, solid-phase analytical device, and an assay method using the device, which is advantageous over devices and assay methods of the prior art. The device of the invention, although applicable to many types of analysis of various fluids, is especially advantageous over the conventional solid-phase immunoassay techniques aforedescribed, for performing immunoassays. Moreover, the device of the invention is comparatively easy to use and less complex by comparison with prior art assay apparatus, and provides the additional advantage of being capable of appropriate treatment, during manufacture, to meet the particular needs of a given assay. The device of the invention has also been found to be highly sensitive and to various analytes, and to possess other advantages which are herein described.

In accordance with the invention, an analytical device for determining the presence or amount of a substance in a fluid test sample comprises a porous, non-bibulous fiber matrix impregnated with a hydrophobic polymer. The fiber matrix is composed of a suitable, non-absorbent material such as glass, and the polymer forms a surface coating on at least a portion of the fibers of the matrix. In a preferred embodiment, the device further comprises a reagent, for example an antigen or antibody to a substance of interest in a sample undergoing analysis, which is capable of reaction with the substance in the sample. The polymer coating of the matrix is capable of retaining the reagent within the matrix, for example, immobilized upon or chemically bound to the polymer.

In addition, the present invention provides a method for performing an assay utilizing the analytical device of the invention. In a preferred embodiment, the method comprises the steps of contacting a sample fluid containing a substance, e.g., antigen or antibody, capable of being bound to the reagent retained within the matrix of the device, removing unbound material, and contacting the matrix with a second reagent capable of becoming bound to the substance which is bound by the reagent retained within the matrix. Thereafter, unbound material is again removed and the device contacted with a third reagent which, in the presence of the second reagent, produces a detectable response which is indicative of the presence and/or amount of the substance in the sample.

In another embodiment of the method of the present invention the second reagent is instead capable of becoming bound to the reagent retained within the matrix. Illustrative of such embodiment is a method wherein the substance in the sample fluid is an antigen and the first reagent retained by the matrix of the device is an antibody and the second reagent is the substance conjugated with an enzyme.

## Brief Description of the Drawings

Fig. 1 is a top plan view of a preferred embodiment of an analytical device in accordance with the present invention.

Fig. 2 is a side view in cross section of the device of Fig. 1, taken along the line 2-2 thereof.

Fig. 3 is a side view in cross section, taken along the line 3-3 of Fig. 4, showing the device of Figs. 1 and 2 mounted in one embodiment of holding apparatus in accordance with the invention, such holding apparatus being adapted for assisting, when the device of the invention is in use, in the assay of fluids for substances contained therein.

Fig. 4 is a perspective view of the device of Figs. 1 and 2 mounted in the holding apparatus illustrated in Fig. 3.

Fig. 5 is a generally schematic side view in cross section showing the preferred device of Figs. 1 and 2 mounted in another embodiment of holding apparatus therefor, which in accordance with the present invention is adapted to enable the device to be utilized in the instrumental determination of the presence and/or amount of substances in a fluid sample under analysis.

Fig. 6 shows another preferred embodiment of the analytical device of the present invention, which includes an additional layer of material having various functions when the device is used in performance of an assay, as described herein.

Fig. 7 shows the removal of the additional layer of material of the device shown in Fig. 6, which removal is preferably effected during the performance of a perferred assay using the device.

Detailed Description of the Invention

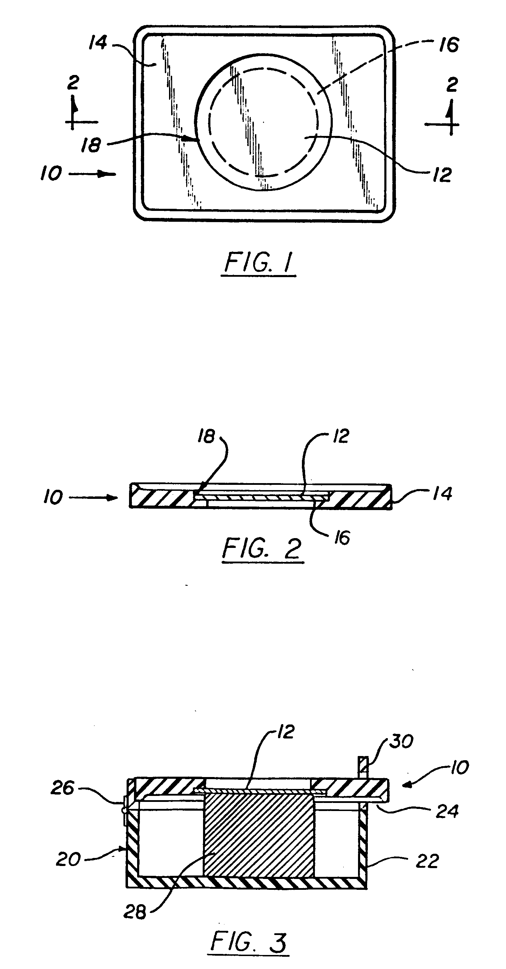

Referring to Figs. 1 and 2 of the drawings, a preferred embodiment of the analytical device of the present invention is shown generally at 10. The device 10 comprises a generally circular, disk-shaped reaction matrix 12, within which matrix 12 various chemical reactions and changes can take place when the device 10 is used (as described, infra, in detail) in the performance of assays for analytes of interest in a fluid undergoing analysis. The structure and composition of the matrix 12 is described in greater detail, infra. The device 10 also comprises a carrier 14 for the matrix 12, which can be made of any suitable material such as plastic, metal or other rigid or semi-rigid substance. In the preferred embodiment shown, the carrier 14 completely surrounds the matrix 12 and functions as a support therefor. In order to accomplish this function, the carrier 14 has a generally circular flange 16 extending into a generally circular cavity 18, which flange 16 serves as a support and surface for attachment (by means of heat sealing or a suitable adhesive, not shown) of the matrix 12 to the carrier 14.

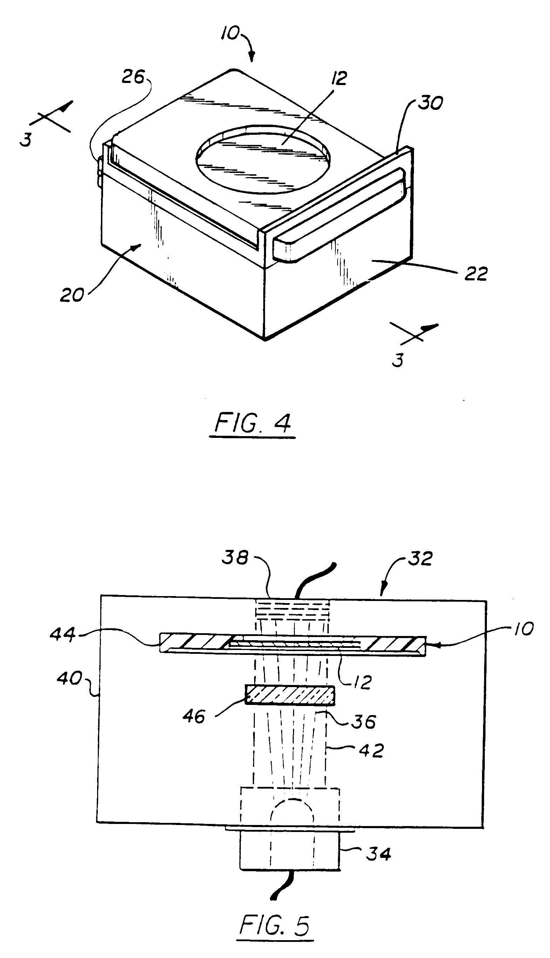

With reference now to Figs. 3 and 4 of the drawings, the analytical device 10 is shown mounted in holding apparatus 20 which, in this preferred embodiment of the invention, facilitates use of the device 10 in the performance of assays of various fluids, as described in more detail infra. The holding apparatus 20 includes a base 22 of a solid material such as plastic or metal, and support means 24 of similar composition integral therewith and adapted for supporting the device 10 in a suitable position for performing an assay. In addition, the apparatus 20 includes a hinge 26 connecting the support means 24 and the base 22 and functioning to enable, when the device 10 is mounted thereupon as shown, of the reaction matrix 12 of the device 10 to be brought into contact, as desired, with a layer of absorbent material 28 which is mounted within the base 22. As will be described hereinafter in more detail, this especially advantageous feature of the apparatus 20 enables excess fluid, during the performance of an assay, to be easily absorbed, as necessary, from the reaction matrix 12 at a particular point or points during the assay procedure. The holding apparatus 20 also includes a positioning bar 30, which assists in proper orientation of the device 10 when the latter is placed in the apparatus 20, during for performance of an assay procedure.

With reference now to Fig. 5 of the drawings, apparatus for instrumentally measuring a detectable response produced to an analyte of interest, during performance of an assay using an analytical device according to the invention (such as the device 10 shown and described with reference to Figs. 1 and 2), is illustrated generally at 32. A particularly advantageous feature of the instrument apparatus 32 is its incorporation therein of source means, shown at 34, for passing radiative energy, for example in the form of a light beam (shown at 36), through the reaction matrix 12 of the device 10; a portion of the light beam 36 emerging after passage through the matrix 12 thus impinges on detector means 38. In the preferred embodiment shown, a housing 40, fabricated of suitable material such as substantially solid metal, is provided as part of the apparatus 32 and includes a cavity 42 extending therethrough within which, at opposite ends thereof, are mounted the radiative energy source means 34 and the detector means 38. The apparatus 32 further includes a mounting slot 44 therein substantially perpendicularly disposed with respect to the cavity 42 and configured so as to receive and to hold therein the device 10, in order to obtain measurement of a detectable response therefrom corresponding to the passage through or absorbance of light by the matrix 12 as a result of chemical and biological reactions and changes which occur therein when an assay is performed. In addition, a conventional optical glass filter 46 is situated in the optical path of the light beam 36, and serves to pass only selected wavelength of light therethrough to the detector 38. Accordingly, the apparatus 32 can be considered a simple spectrophotometer. For example, if the detectable response in the matrix 12 produced by the reactions and changes produced during a particular assay is one wherein a color is developed in the matrix 12, and wherein increasing color development indicates an increasing level of a particular analyte in a test sample undergoing analysis, then a diminishing level of light passing through the matrix 12 and impinging upon the detector means 38 (i.e., more light is absorbed by the matrix 12), corresponds to that increased level of analyte in the sample. The interpretation of such results produced by the action of the light impinging on the detector means 38 is capable of being accomplished in ways well known to those skilled in the art, such as by conversion of analog signals generated, when the detector 38 is a conventional photodiode, to digital information using largely conventional electronics. Such electronics are well known to those skilled in the art and are capable of producing a digital, human-readable signal corresponding to the presence and/or amount of analyte present in the test sample.

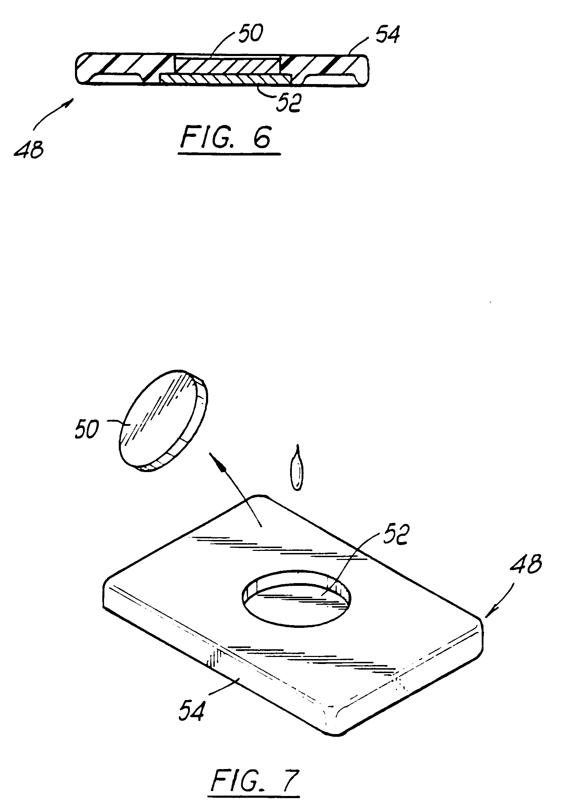

Referring now in more detail to Fig. 6 of the drawings, another preferred embodiment of the analytical device of the invention is illustrated generally at 48. The device 48 is generally similar to the device 10 shown and described in reference to Fig. 1, however, the device 48 further comprises an upper layer, or

4

"overlayer" of material 50 disposed over the reaction matrix 52 thereof. The "overlayer" 50 is composed of a fibrous material such as glass or cellulose filter paper, and which, when the device 48 is used to perform an assay, can perform various functions. Depending upon the type of assay being performed, the overlayer can perform such functions as a reservoir to retain sample over the reaction matrix; as a vehicle to retain reagents, e.g., lyophilized reagents, to be used in an assay; as a "prefilter" to remove extraneous particulate matter in a sample, or, for example, to separate and to hold blood cells from a whole blood sample while allowing plasma to pads through. In addition, during performance of an assay using the device 48, the "overlayer" matrix 50 can be removed, as desired, during a step of the assay in order to remove material which may be retained therein, or for other purposes such as to expose the reaction matrix 52. Such removal is illustrated in Fig. 7. The reaction matrix 52 of the device 48, and the holder 54 therefor are of substantially identical construction, and serve substantially the same function as previously described for the reaction in matrix 12 and holder 14 of the device 10 of Fig. 1. In accordance with the invention, an analytical device comprises a porous, non-bibulous fiber matrix impregnated with a hydrophobic polymer. By "porous" is meant that the matrix is composed of a material into and through which fluids can easily pass. By "non-bibulous" is meant a fiber matrix material which is incapable or substantial absorption of water or aqueous solutions. In devices of the present invention, properties of porosity, and non-bibulous qualities, can be achieved simply by selection of an appropriate material for the reaction matrix material, such as glass, cellulose, nylon and other synthetic fibrous materials well known to those skilled in the art. In addition, since according to a preferred embodiment of the invention the fiber matrix is impregnated, in at least a portion thereof, with a hydrophobic polymer, such hydrophobic properties can be derived from selection of the polymer. Thus, suitable polymers for impregnating the preparation of devices according to the invention include, for example, polystyrene, polymethylacrylate, polypropylene, polyacrylonitrile and polycarbonate.

Accordingly, in the preparation of a preferred analytical device in accordance with the present invention, a suitable polymer, such as one selected from the aforedescribed, is dissolved in an organic solvent, for example acetone, benzene or xylene. It is preferred that the concentration of polymer in the solvent be within the range of from about 0.1 to 100 milligrams per milliliter (mg/ml). The fibrous matrix material, which can be selected, for example, from those aforedescribed is cut or otherwise formed to the shape desired (in the preferred embodiments hereof, substantially circular; however, it is to be appreciated that both the shape and dimensions of the matrix can vary widely, as can the shape and dimensions of the holder therefor). Thereafter, the formed matrix material is positioned appropriately over the hole therefor in the holder, and attached thereto using well-known means such as adhesive bonding or heat sealing, depending upon the composition of the holder. A small amount (in the usual case between about 1 and 250 microliters (ul) of the polymer solution is applied per square centimeter of the matrix. The solvent portion of the polymer solution is then allowed to evaporate or is driven off, usually at a temperature between about 20 and 40 degrees C, for between approximately 0.5 to 24 hours. This serves to impregnate the polymer onto at least a portion of the surfaces of the fibers in the matrix; substantially complete impregnation is preferred, but partial impregnation is usually sufficient to enable the device to perform satisfactorily in an assay procedure. In an alternative approach, these latter steps can be used to also bind the matrix to the holder, eliminating the need for adhesive or heat-bonding of the matrix to secure it to the holder.

Following the foregoing procedures, the preferred device is ready to be contacted with a reagent which is retained therewithin during the subsequent performance of an assay using the device. In a preferred technique, an antibody or antigen (in instances where it is intended that the device be utilized in immunoassays) is applied to the matrix; the antibody or antigen preferably has a concentration within the range of about 10 to 10,000 micrograms per milliliter (ug/ml), and is applied to the matrix in a volume of about 1-250 microliters per square centimeter of filter area. Then, the device is incubated at between about 20 and 50 degrees C for a period of approximately 2 to 18 hours. Excess fluid is thereafter removed by blotting with a suitably absorbent material, such as a cellulosic absorbent layer of material which can be manually manipulated into contact with the matrix or, when apparatus such as that shown in Figs. 3 and 4 is used, can be part of a holder for the device. In the preparation of devices of the invention, this cycle of washing with a buffer and blotting can be repeated many times, depending upon the intended use of the device and the necessity, if the device is to be used in a particular assay, of devices prepared for use in that assay necessarily being largely free of unbound antibody or antigen prior to conduct of the assay procedure. In this manner, devices of the invention can be specifically and especially prepared with the specificity and sensitivity requirements of a particular assay taken into account.

The device provided by the invention, it is to be appreciated, can be advantageously employed in a wide variety of otherwise well-known assay techniques and procedures, and is not limited in application to the specific immunoassay techniques described in detail herein. The device can thus be used in so-called "competitive binding" assays or similar procedures; in addition, it can be employed in other assays such as typical enzyme assays for such analytes as glucose, uric acid or the like, which are not immunoassays but which can advantageously be carries out by first retaining at least one reagent used in such assays within the reaction matrix of the device of the invention. It will be readily apparent to those skilled in the analytical arts that the instant analytical device can be profitably applied to a wide variety of uses in various types of assay procedures, and thus is in no way limited to the details disclosed herein.

Analytical devices produced in accordance with the principles of the instant invention are, however, especially advantageously adapted to be used to perform enzyme immunoassays, particularly so-call "sandwich" enzyme immunoassay, procedures. Such devices are capable of enabling such assays to be performed relatively more simply than typical "bead" or other assays of the prior art which require much more elaborate, time-consuming and costly equipment and materials, and as well have been found to be capable of surprising sensitivity. A generalized example for a preferred "sandwich" immunoassay procedure utilizing a device of the invention is as follows:

Step a) Retention of antibody or antigen in the reaction matrix of the device, as previously described;

Step b) Application of a test sample containing antigen or antibody to be determined to the matrix;

Step c) Application of a enzyme-conjugated antibody or antigen to the antigen or antibody of Step b);

Step d) Washing, to remove unbound material; and

Step e) Application of a chromophore which, in the presence of the enzyme portion of the conjugate of Step d), produced a detectable color response.

A more detailed discussion of how such "sandwich" assay procedures can advantageously be carried out using the device of the present invention is set forth in the Examples, infra.

In accordance with the present invention, the analytical device is capable of producing a detectable response which is indicative of the presence and/or amount of a substance in a fluid under analysis. Such a detectable response, in preferred embodiments of the invention, can be color development, through the use of a chromogen as the third reagent, in the reaction matrix of the device following a series of assay steps, such as those previously described, or it can be any number of responses well known in the analytical art and used for similar purposes. For example, the response produced by the device can be one of fluorescence, provided appropriate reagents are employed in the assay, as is well known to those skilled in the art. The response can be also chemiluminescence, or any of a variety of radiative energy responses detectable either visually by the human eye, or instrumentally by various known equipment. Thus, it is to be especially appreciated that in use of the device of the invention, many different types of detectable responses are possible and desirable and the inventive concepts are not to be limited thereby.

## EXAMPLES

The following Examples illustrate preferred ways of making and using the analytical device of the invention, as well as assay procedures utilizing holding apparatus and apparatus for determining a detectable response produced by the device to an analyte in a test sample, such as has been aforedescribed and shown in the drawings. Accordingly, the Examples are intended to be only illustrative, and in no way to be construed as limitative of the scope of the present invention, which scope is defined solely by the appended claims.

Unless otherwise indicated, all percentages expressed herein are by weight.

### Example 1: Fabrication of Analytical Devices of the Invention

Analytical devices having substantially the overall shape and appearance of the device shown and described with reference to Figs. 1 and 2 were prepared according to the following procedure.

To a 250-milliliter (ml) Erlenmyer flask with a glass stopcock were added 100 ml xylene and 440 milligrams (mg) polystyrene resin (Type R5, commercially available from Amoco). The mixture in the flask was stirred using a magnetic stirrer, at room temperature, until the resin was observed visually to be completely dissolved. Substantially circular disks, having a diameter of about 15.5 millimeters (mm), were then punched out of a sheet of glass fiber filter papers (Whatman 934/AH) with a #11 cork borer. A number of these disks were next positioned over injection-molded plastic holders having the overall shape and appearance of the holder 14 of the device 10 shown and described with reference to Figs. 1 and 2. The holders used in this particular case measured approximately 3.5 cm (1-3/8 inches) in width by 4.8 cm (1-7/8 inches) length, and had an overall thickness of approximately 0.3 cm (1/8 inch), with a substantially centrally-located, circular hole extending therethrough about 14.5 mm in diameter (over which hole the disks were positioned). The holders, with the disks positioned thereupon, were then placed in a fume hood and 90 microliters (ul) of the polystyrene mixture previously described were added to each disk, and the disk/holder assemblies allowed to stand under covers for between about 5 to 8 minutes. This procedure caused the disks to become bonded to the plastic holders, by action of the xylene solvent in the mixture, around the periphery of the disks and of the holes in the holders; the disks were noted to be securely attached to the holders after removal of the coverings and overnight evaporation of the solvent. The foregoing procedure thus describes the fabrication of devices of the invention, with the disks constituting the reaction matrix of the devices.

Example 2: Enzyme Immunoassay (EIA) for Hepatitis B Surface Antigen (HBsAg)

Analytical devices which had been prepared in accordance with the instant invention as described in Example 1 were utilized to determine HBsAg in specimens of human blood serum, according to the following assay procedure. This Example particularly demonstrates the performance of such devices in one type of a so-called "sandwich" enzyme immunoassay.

Partially purified guinea pig anti-HBsAg was diluted to 100 micrograms (ug) per ml in 50 millimolar (mM) sodium carbonate, pH 9.6. Forty ul of this antibody solution was then added to the reaction matrix area of a device prepared as previously described in Example 1. The device was then covered and sealed with "Parafilm®" (commercially available from Scientific Products), whereafter it was allowed to stand at room temperature overnight. The Parafilm® was then removed and the matrix area of the device was blotted by contacting its bottom side with a stack of water-absorbing paper (commercially available from Scott Paper Company), in order to remove excess fluid from the device. Then, 20 ul of a 2 % bovine serum albumin solution in phosphate buffered saline were added to the matrix area, and the device incubated for about 30 minutes at 40 degrees C in a moist chamber. The matrix area was again blotted to remove excess fluid, as previously described, and 25 ul of phosphate buffered saline containing .05 % Tween® 20 ("PBS/Tween®", commercially available from Sigma Chemical Co.), was added. This latter "blot/add" cycle was repeated seven more times, and thereafter the device was deemed to be ready for the performance of the assay steps following. About twenty ul of a sample, comprising human blood serum, was added to the matrix area of the device. The device with the sample thereon was then incubated, substantially as previously described, for a total of about 5 minutes. The matrix area was then blotted to remove excess fluid, as aforedescribed. The matrix was then washed with an application of phosphate buffered saline/Tween® (PBS/Tween®) solution seven separate times. To the washed matrix were added 20 ul of a horseradish peroxidase("HRPO")-antibody conjugate, which had been previously prepared by the well-known method of periodate oxidation and borohydride reduction, generally as described by NaKane, P.K. and Kawaoi, A., in J. Histochem. Cytochem., vol. 22, p. 1084 (1974). After addition of the conjugate, the device was again incubated for a period of 30 minutes at 40 degrees C., and thereafter the matrix was washed again with PBS/Tween®.

A chromogen solution was prepared which contained 0.0175 % tetramethylbenzidine (TMB), 1.0 % acetic acid (HOAc), 2.02 % sodium phosphate (tribasic), 0.98 % citric acid and 0.02 % hydrogen peroxide (v/v) at pH 5.5. Twenty ul of the chromogen solution were then added to the matrix of the device, with the result that a blue color began to develop virtually immediately in the matrix. The color development in the matrix was read visually, as an indication of the presence of HBsAg in the original sample.

Example 3: Assay for Human Chorionic Gonadotropin (HCG)

Forty ul of purified goat anti-HCG antibody, obtained using conventional procedures by immunizing a goat with beta-HCG, at 100 ug per ml in 50 mM sodium carbonate, pH 9.6, was added to the reaction matrix of a device of the invention which had been prepared as described in Example 1. The device was then incubated at room temperature overnight. Following blotting as described in Example 2, 20 ul of 2 % bovine serum albumin were added to the matrix and the device again incubated for a period of about 30 minutes at 40 degrees C. The matrix was thereafter blotted and washed seven times with PBS/Tween® as described in Example 2. Twenty ul of a human serum sample were then added to the matrix, and the device incubated for 5 minutes at 40 degrees C. The matrix was again blotted to remove excess fluid and the sample addition, incubation and blotting cycles repeated 5 more times, and the matrix was thereafter washed seven times.

Twenty ul of an enzyme-anti-HCG conjugate, obtained by the method described in Example 2, were added to the matrix of the device and, after further incubation for 15 minutes at 40 degrees C, the matrix was again blotted. A second aliquot of the foregoing conjugate was then added, following by another incubation and washing step, as previously described. Color development in the matrix was then induced by the addition of 20 ul of the aforedescribed TMB solution, and the color read visually as an indication of the presence of HCG in the test sample.

Example 4: "Sandwich" EIA for HBsAg; Simultaneous Mode

Another form of assay of human blood serum using the device provided by the invention was carried out by mixing a portion of the conjugate described in Example 2 with the test sample, prior to application of the sample to the reaction matrix of the device. Thus, the procedure was carried out substantially as described in Example 2, except that in this case the steps of sample incubation and conjugate incubation were combined. For immobilization of the antibody in the reaction matrix, in this experiment mixtures of monoclonal anti-HBsAg, obtained by conventional, well-known hybridoma methodology, were diluted to a concentration of 100 ug/ml with 50 mM sodium carbonate, pH 9.6. The monoclonal antibody solution was then added to the matrix, as has been described. After treatment with 2 % bovine serum albumin and a washing step (with PBS/Tween®), a 1:1 mixture of sample and conjugate was added to the filter in five 20 ul portions every 3 minutes, over a total of fifteen minutes. During each 3 minute interval, the matrix was incubated at room temperature and blotted as aforedescribed at the end of each interval The matrix was thereafter again washed with PBS/Tween®, the aforedescribed TMB chromogen solution was added and color development observed as described in Example 2, indicating the presence of HBsAg in the sample.

Example 5: "Sandwich" EIA for HBsAg; Utilization of an "Overlayer" in a Preferred Service of the Invention

Another assay was conducted using a further preferred embodiment of the analytical device of the invention, which device had been constructed substantially as illustrated and described in connection with Figs. 6 and 7 of the drawings. The particular device was one wherein a top, or "overlayer" of material was utilized, in this particular instance a circular piece of glass fiber matrix material (comprising 3 layers, each 1 mm in thickness and 11 mm in diameter, of Whatman GF/D filter paper, and having substantially the same shape as the reaction matrix of the device). The "overlayer" was employed as a "reservoir", or temporary "trap", for the test sample undergoing analysis for the presence of HBsAg therein.

To perform this assay, 300 ul of a 1:1 mixture of sample and conjugate, as described infra was added to the "overlayer" matrix in a single application. Then, for 5 successive 3 minute intervals, portions of the fluid retained in the "overlayer" matrix were pulled into the reaction matrix of the device by blotting as described in the prior Examples. The contact time of the matrix with the blotting material was carried out in a sequence of 0.5, 1, 2, 4 and 8 seconds. This procedure was found advantageously to afford a somewhat constant flow of fluid across the reaction matrix during each blotting. The remainder of the assay was performed virtually identically to that of Example 4.

Example 6: "Sandwich" EIA for HBsAg, Utilizing Lyophilized Conjugate in the "Overlayer"

An assay was performed using the device and procedure substantially as described in Example 5, except in this case the conjugate was previously lyophilized with a bench top lyophilizer, commercially available from Virtis Co., while incorporated in the "overlayer" matrix. In performance of this assay, however, 150 ul of the test sample was added to the "overlayer" matrix which contained the conjugate. Accordingly, this step of the assay served to reconstitute the conjugate, and also eliminated the necessity of mixing of the sample with the conjugate. Furthermore, carrying out the assay according to this preferred procedure confers greater stability for the conjugate prior to performing the assay since, as is well known in the art, such conjugates are intrinsically more stable in lyophilized form than when maintained in liquid form. As previously stated, the remainder of the assay procedure was performed similarly to that of Example 5, with sequential blotting, washing, chromogen addition and reading steps as set forth therein.

Example 7: "Inhibition" Assay for Antibody to Hepatitis B Core Antigen (HBcAg)

In this assay configuration, performed using an analytical device according to the invention as described in Example 6 and in reference to Figs. 6 and 7, the antigen was immobilized within the reaction matrix of the device. Antibody in the sample then was allowed to compete with the antibody associated with the conjugate for binding to the solid phase antigen. Thus, the level of antibody in the sample correlate inversely with the amount of the conjugate which becomes bound to the solid phase during the assay.

A recombinantly-engineered core antigen, which had been produced by conventional genetic engineering techniques, was diluted to a level of 100 ug/ml with 50 mM sodium carbonate, pH 9.6. To the reaction matrix of the device were added 40 ul of the antigen solution. The device with the antigen solution so applied was then incubated at room temperature overnight, after which the reaction matrix was blotted as aforedescribed and the device again incubated with 20 ul of 2 % bovine serum albumin in phosphate buffered saline, at 40 degrees C for approximately 30 minutes. The device was then washed with PBS/Tween® as in the previous Examples.

A test sample of human serum was next mixed in a ratio of 1:2 with an anti-HBcAg HRPO conjugate. To the "overlayer" matrix of the device, which in this instance functioned as a reservoir as previously described, was added 300 ul of the mixture. The reaction matrix of the device was then blotted every 3 minutes, for a total of 15 minutes. The duration of each blotting was 0.5, 1, 2, 4 and 8 seconds, respectively. The device was thereafter washed as aforedescribed, color developed with TMB and read visually as previously described.

### Example 8: "Sandwich" Assay for Streptococcus A Antibody

Purified Goat anti-Strep A antibody which, had been conventionally raised in a goat with Strep A lipopolysaccharide, was diluted to 200 ug/ml in 50 mM sodium carbonate, pH 9.6. To the reaction matrix of a device which had been produced according to the invention as described in Example 1, were added 40 ul of the antibody solution. The device was then sealed with Parafilm®, as previously described, and incubated at room temperature overnight. The reaction matrix was then blotted as aforedescribed and 20 ul of 2 % bovine serum albumin in PBS was applied thereto, whereafter the device was again incubated, at 40 degrees C for 30 minutes, and washed as described in the previous Examples.

Twenty ul of a sample, which included a mutanolysin-treated, cultured Strep A organism at a level of $5 \times 10^3$ organisms per ml, were added to the reaction matrix of the device, and the device incubated for 5 minutes at 40 degrees C. The reaction matrix was again blotted from underneath, and this sample addition, incubation and blotting cycle repeated 5 more times, after which further washing was performed. Twenty ul of purified rabbit anti-Strep A antibody (raised in a rabbit with Strep A lipopolysaccharide, 10 ug/ml) was then added, and the device incubated at 40 degrees C for 15 minutes, after which it was washed for 15 cycles. Finally, 20 ul of goat anti-rabbit:horseradish peroxidase produced by the methodology previously described, was applied to the reaction matrix and the device incubated for another 15 minutes. Following another 14 wash cycles, the device was color developed with TMB as aforedescribed and read visually in the manner previously stated.

### Example 9: "Sandwich" EIA for N. Gonorrhoeae

A device in accordance with the invention was produced as described in Example 1, except that the glass fiber material was first coated with polystyrene as described therein at a concentration of 28 mg/ml. Thereafter, 25 ul of a deoxycholate-treated sample of N. Gonorrhoeae were added to the reaction matrix, and the device incubated at 40 degrees C for 5 minutes. Excess fluid was then blotted from the device as aforedescribed and the sample addition, incubation and blotting cycles repeated 6 additional times. The device was then washed, also as previously described, with PBS/Tween for 7 separate cycles. Twenty-five ul of rabbit anti-Gonorrhoeae which had been raised in rabbit with N. Gonorrhoeae, were then added to the reaction matrix and the device again incubated, at 40 degrees C for 20 minutes, followed by a PBS/Tween® wash for 14 cycles. Then, 40 ul of goat anti-rabbit:HRPO (Kirkegaard & Perry, Inc.) were added and incubated at 40 degrees C for 20 minutes, and subsequently washed in PBS/Tween for 14 cycles. Color development of the device and visual reading were then accomplished as described in the prior Examples.

### Example 10: Fabrication of Devices of the Invention; Another Procedure

Additional analytical devices according to the invention were prepared using the following procedure, rather than the procedure described in Example 1.

An 8 inch by 10 inch sheet of Whatman GF/D filter paper was dipped into a bath of tetrahydrofuran containing 4.4 mg/ml of polystyrene (TYPE R5 from Amoco). The dipped sheet was then removed from the bath with a steel screen and air-dried in a fume hood for approximately 2 hours at room temperature. Substantially circular disks, approximately 16 mm in diameter were then punched out of the sheet using a conventional die-cutting device. These disks were then placed over the centrally-located, slightly smaller circular hole of holders having substantially the overall appearance of the holder 14 shown and described in reference to Figs. 1 and 2 except for a ridge of about 2 mm height rising along the circumference of the hole 18 therein. The disks were bonded to the holders by means of heat sealing around their peripheries to the ridge along the circumference of the holes in the holders using conventional heat sealing apparatus, thereby forming reaction matrices on each of the holders.

Example 11: Assay for Whole Blood Alpha Fetoprotein (AFP)

Devices prepared in accordance with the invention as described in Example 10 were utilized to assay for AFP in a sample of human whole blood. A monoclonal anti-AFP antibody which had been conventially produced and purified was diluted to 100 ug/ml with 50 mM sodium carbonate, pH 9.6. To the devices were then added 150 ul of the diluted antibody solution. The devices were then incubated overnight at room temperature, after which they were blotted from underneath their reaction matrices, as previously described. Fifty ul of 2 % BSA/PBS were then added to the reaction matrices and the devices incubated at 40 degrees C for about 1 hour, whereafter they were washed, as previously described, with PBS/Tween®.

A number of approximately 12 mm diameter disks were punched out of a sheet of Whatman GF/B filter paper, using the technique previously described. These disks were then placed directly over the reaction matrices of the devices, and, to facilitate surface contact, both the disks and the reaction matrices of the devices were wet with anti-AFP:HRPO conjugate. The assay was then continued by adding 20 ul of human whole blood to the filter disks, which now constituted an upper, "overlayer" matrix of the devices. Each of the devices was next placed in holding apparatus, substantially as shown and described with reference to Figs. 3 and 4 of the drawings, and the reaction matrices of the devices held down against blotting material, substantially as shown at 28 of Fig. 3, which was mounted in the holding apparatus. After the blood had soaked through the "overlayer" matrix, a time of about 5 seconds, the conjugate, in an amount of about 75 ul, was added to the "overlayer" over a period of about 1 to 2 seconds. Thereafter, the devices were incubated at room temperature for about 10 minutes; the "overlayer" matrices were removed, and the reaction matrices of the devices were washed with PBS/Tween®. TMB, in an amount of 50 ul, was then added and color development proceeded as described in the preceding Examples, which was read visually.

Example 12: Quantitative Assay of AFP

An assay was conducted substantially as described in Example 11, wherein reaction matrices of devices prepared as therein described were similarily coated with monoclonal AFP antibody. However, in this assay procedure, 40 ul of standard solutions containing AFP levels of 0, 25, 100, 250 and 500 nanograms per ml were added to each device. These additions were followed immediately by the addition of 40 ul of anti-AFP:HRPO conjugate. The devices were allowed to stand for 30 minutes at room temperature, and then washed with PBS/Tween®. Color development proceeded with TMB, as previously described. This color development was read with a microtiter transmittance reader available commercially from Bio-Tek Instruments, Inc. The results produced by instrumental absorbance readings taken by the transmittance reader from the devices are set forth in the following table, representing color development after two minutes.

| STANDARDS (ng/ml) | 0 | 25 | 100 | 250 | 500 |
|---|---|---|---|---|---|
| ABSORBANCE (at 650 nm) | 0.036 | 0.230 | 0.682 | 0.699 | 0.762 |

The foregoing data shows that devices according to the invention can be used for quantitative assays as well as qualitative assays, and can produce data useful for constructing, e.g., a calibration curve for an instrument, such as the transmittance reader previously described. Observed reading from actual test samples can then be compared against the calibration curve. The interpolated value of the AFP concentration thus provides and estimation of the AFP level which was present in the sample. It is apparent that similar readings could also be obtained by using, for example, the apparatus shown and described with reference to Fig. 5 of the drawings.

**Claims**

1.  An analytical device for determining the presence or amount of a substance in a fluid test sample, said device having a support coated with a polymer retaining a reagent capable of reaction with a substance in the sample, characterized in that the support comprises a porous, non-bibulous fiber matrix impregnated with a hydrophobic polymer forming a surface coating on at least a portion of the fibers of the matrix.

2. The analytical device set forth in Claim 1, wherein the hydrophobic polymer is selected from the group consisting of polystyrene, polymethylacrylate, polypropylene, polyacrylonitrile and polycarbonate.

3. The analytical device set forth in Claim 1, wherein the matrix is composed of glass fibers.

4. The analytical device set forth in Claim 1, wherein the reagent is an antigen.

5. The analytical device set forth in Claim 1, wherein the reagent is an antibody.

6. The analytical device set forth in Claim 1, wherein the device additionally comprises a removable matrix of material overlaying said fiber matrix.

7. A method for determining the presence or amount of a substance in a sample fluid, comprising the steps of
a) applying a portion of the sample fluid to the support of the device of claim 1;
b) applying a second reagent capable of reacting with the first reagent retained by said support;
c) applying a third reagent capable of producing at said support a detectable response to the presence of said second reagent of step b); and
d) interpreting the detectable response as an indication of the presence or amount of the substance in the fluid.

8. The method set forth in Claim 7, wherein the substance in the sample fluid is an antigen and the first reagent retained by said support of the device is an antibody and the second reagent of step b) is the substance conjugated with an enzyme.

9. A method for determining the presence or amount of a substance in a sample fluid, comprising the steps of
a) applying a portion of the sample fluid to the support of the device of claim 1;
b) applying a second reagent capable of reacting with the substance from the sample fluid which has been retained by said support through reaction with said first reagent;
c) applying a third reagent capable of producing at said support a detectable response to the presence of said second reagent of step b); and
d) interpreting the detectable response as an indication of the presence or amount of the substance in the fluid.

10. The method set forth in Claim 9, wherein the substance in the sample fluid is an antibody or antigen; the first reagent retained by said support of the device is an antigen or antibody capable of reacting with the substance; and the second reagent of step b) is enzyme conjugated to an antibody or antigen reactive with the substance.

11. The method set forth in Claim 7 or 9, wherein the third reagent capable of producing a detectable response is a chromogen, and the detectable response is color development.

**Revendications**

1. Dispositif d'analyse destiné à la détermination de la présence ou de la quantité d'une substance dans un échantillon liquide à doser, ledit dispositif possédant un support revêtu d'un polymère retenant un réactif capable de réagir avec une substance dans l'échantillon, caractérisé en ce que le support comprend une matrice fibreuse poreuse, non hydrophile, imprégnée d'un polymère hydrophobe formant un revêtement superficiel sur au moins une partie des fibres de la matrice.

2. Dispositif d'analyse selon la revendication 1, dans lequel le polymère hydrophobe est sélectionné dans le groupe constitué par le polystyrène, le polyméthylacrylate, le polypropylène, le polyacrylonitrile et le polycarbonate.

3. Dispositif d'analyse selon la revendication 1, dans lequel la matrice est composée de fibres de verre.

4. Dispositif d'analyse selon la revendication 1, dans lequel le réactif est un antigène.

EP 0 186 100 B1

**5.** Dispositif d'analyse selon la revendication 1, dans lequel le réactif est un anticorps.

**6.** Dispositif d'analyse selon la revendication 1, dans lequel le dispositif comprend additionnellement une matrice amovible en un matériau recouvrant ladite matrice de fibres.

**7.** Procédé de détermination de la présence ou de la quantité d'une substance dans un échantillon liquide, comprenant les étapes de :

a) application d'une partie de l'échantillon liquide sur le support du dispositif selon la revendication 1 ;

b) application d'un second réactif capable de réagir avec le premier réactif retenu par ledit support ;

c) application d'un troisième réactif capable de produire sur ledit support une réaction détectable à la présence dudit second réactif de l'étape b) ; et

d) interprétation de la réaction détectable comme étant l'indication de la présence ou de la quantité de la substance dans le liquide.

**8.** Procédé selon la revendication 7, dans lequel la substance dans l'échantillon liquide est un antigène, le premier réactif retenu par ledit support du dispositif est un anticorps et le second réactif de l'étape b) est la substance conjuguée avec une enzyme.

**9.** Procédé de détermination de la présence ou de la quantité d'une substance dans un échantillon liquide, comprenant les étapes de :

a) application d'une partie de l'échantillon liquide sur le support du dispositif selon la revendication 1 ;

b) application d'un second réactif capable de réagir avec la substance de l'échantillon liquide retenu par ledit support durant la réaction avec ledit premier réactif ;

c) application d'un troisième réactif capable de produire sur ledit support une réaction détectable à la présence dudit second réactif de l'étape b); et

d) interprétation de la réaction détectable comme étant l'indication de la présence ou de la quantité de la substance dans le liquide.

**10.** Procédé selon la revendication 9, dans lequel la substance dans l'échantillon liquide est un anticorps ou un antigène ; le premier réactif retenu par ledit support du dispositif est un antigène ou un anticorps capable de réagir avec la substance ; et le second réactif de l'étape est une enzyme conjuguée à un anticorps ou à un antigène réagissant avec la substance.

**11.** Procédé selon la revendication 7 ou 9, dans lequel le troisième réactif capable de produire une réaction détectable est un chromogène et la réaction détectable est la formation d'une coloration.

**Patentansprüche**

**1.** Analytische Vorrichtung zur Bestimmung der Gegenwart oder Menge einer Substanz in einer fluiden Testprobe, welche Vorrichtung einen mit einem Polymer beschichteten Träger aufweist, das ein mit einer Substanz in der Probe reaktionsfähiges Reagens zurückhält, dadurch gekennzeichnet, daß der Träger eine poröse, nicht-saugende Fasermatrix umfaßt, die mit einem hydrophoben Polymer imprägniert ist, das eine Oberflächenbeschichtung auf wenigstens einem Teil der Fasern der Matrix bildet.

**2.** Analytische Vorrichtung nach Anspruch 1, worin das hydrophobe Polymer aus der aus Polystyrol, Polymethylacrylat, Polypropylen, Polyacrylnitril und Polycarbonat bestehenden Gruppe ausgewählt ist.

**3.** Analytische Vorrichtung nach Anspruch 1, worin die Matrix aus Glasfasern zusammengesetzt ist.

**4.** Analytische Vorrichtung nach Anspruch 1, worin das Reagens ein Antigen ist.

**5.** Analytische Vorrichtung nach Anspruch 1, worin das Reagens ein Antikörper ist.

**6.** Analytische Vorrichtung nach Anspruch 1, worin die Vorrichtung zusätzlich eine entfernbare Matrix aus Material umfaßt, das die Fasermatrix überdeckt.

12

**7.** Verfahren zur Bestimmung der Gegenwart oder Menge einer Substanz in einem Probenfluid, welches als Schritte umfaßt:

a) die Anwendung eines Teils des Probenfluids auf den Träger der Vorrichtung nach Anspruch 1;

b) die Anwendung eines zweiten Reagens, das mit dem im Träger zurückgehaltenen ersten Reagens reaktionsfähig ist;

c) die Anwendung eines dritten Reagens, das an dem Träger auf die Gegenwart des zweiten Reagens von Schritt b) eine nachweisbare Antwort erzeugen kann; und

d) die Interpretation der nachweisbaren Antwort als Indikator für die Gegenwart oder Menge der Substanz in dem Fluid.

**8.** Verfahren nach Anspruch 7, worin die Substanz in dem Probenfluid ein Antigen ist und das erste von dem Träger der Vorrichtung zurückgehaltene Reagens ein Antikörper ist und das zweite Reagens von Schritt b) die mit einem Enzym konjugierte Substanz ist.

**9.** Verfahren zur Bestimmung der Gegenwart oder Menge einer Substanz in einem Probenfluid, welches als Schritte umfaßt:

a) die Anwendung eines Teils des Probenfluids auf den Träger der Vorrichtung nach Anspruch 1;

b) die Anwendung eines zweiten Reagens, das mit der Substanz aus dem Probenfluid, das durch Reaktion mit dem ersten Reagens auf dem Träger zurückgehalten worden ist, reagieren kann;

c) die Anwendung eines dritten Reagens, die an dem Träger in Gegenwart des zweiten Reagens aus Schritt b) eine nachweisbare Antwort erzeugen kann; und

d) die Interpretation der nachweisbaren Antwort als Indikator für die Gegenwart oder Menge der Substanz in dem Fluid.

**10.** Verfahren nach Anspruch 9, worin die Substanz in dem Probenfluid ein Antikörper oder ein Antigen ist, das an dem Träger der Vorrichtung zurückgehaltene erste Reagens ein Antigen oder Antikörper ist, das oder dar mit der Substanz reagieren kann; und das zweite Reagens aus Schritt b) ein an einen Antikörper oder Antigen konjugiertes Enzym ist, der oder das mit der Substanz reaktiv ist.

**11.** Verfahren nach Anspruch 7 oder 9, worin das dritte Reagens, das eine nachweisbare Antwort erzeugen kann, ein Chromogen ist und die nachweisbare Antwort eine Farbentwicklung ist.

FIG. 1

FIG. 2

FIG. 3

_FIG. 4_

_FIG. 5_

_FIG. 6_

_FIG. 7_